# EUROPEAN PATENT APPLICATION

(11) **EP 4 327 843 A1**
(43) Date of publication of application: **28.02.2024**
(21) Application number: 22832550.2
(22) Date of filing: 30.03.2022
(51) Int. Cl.: A61M 1/16, A61M 1/36

(54) **BLOOD PURIFICATION SYSTEM**

(30) Priority: 01.07.2021 JP 2021110365
(71) Applicant: Nikkiso Company Limited, Tokyo 150-6022 (JP)
(72) Inventor: KAWARABAYASHI, Satoru, Tokyo 150-6022 (JP); TOYODA, Masahiro, Makinohara-shi, Shizuoka 421-0496 (JP); TANAKA, Kensaku, Makinohara-shi, Shizuoka 421-0496 (JP)
(74) Representative: von Hirschhausen, Helge
(86) International application number: PCT/JP2022/016295
(87) International publication number: WO 2023/276389

(57) **Abstract**

To provide a blood purification apparatus configured such that a B-drug solution used in a blood purification treatment process is also utilized as an alkaline chemical in a defatting washing process, and such that assured and smooth performance of the defatting washing process is achieved. A blood purification apparatus includes a line section, a duplex pump (1), and a control unit (13). The line section includes a dialysate introduction line (L1) and a drain-liquid discharge line (L2). The control unit (13) is configured to execute a blood purification treatment process and a defatting washing process. The blood purification treatment process is executed when blood purification treatment is performed in which dialysate is delivered to a dialyzer (C). The dialysate is prepared by diluting an A-drug solution and a B-drug solution to predetermined concentrations. The defatting washing process is executed when defatting washing is performed in which oil and fat in the line section is removed by causing a sodium carbonate solution obtained from the B-drug solution introduced into the line section to flow through the line section. The control unit (13) uses a heating unit (4) and a deaerator (5) when executing a bicarbonate-solution-generating process in which the sodium carbonate solution is generated by a predetermined amount while carbon dioxide is discharged, and when executing, after the bicarbonate-solution-generating process, the defatting washing process in which the sodium carbonate solution is used.

## Description

### Technical Field

The present invention relates to a blood purification apparatus configured to purify a patient's blood by causing the blood to extracorporeally circulate through a blood purifier and a blood circuit.

### Background Art

A dialysis apparatus serving as a blood purification apparatus intended for dialysis treatment or the like typically includes a line section and a delivery unit. The line section includes a dialysate introduction line through which dialysate is to be introduced into a blood purifier, and a drain-liquid discharge line through which drain liquid is to be discharged from the blood purifier. The delivery unit is configured to deliver the dialysate and the drain liquid in the line section. Furthermore, a blood circuit through which a patient's blood is caused to extracorporeally circulate is connected to the blood purifier. While the patient's blood is caused to extracorporeally circulate through the blood circuit, dialysis treatment (blood purification treatment) is performed by using the blood purifier.

In general, such a blood purification apparatus is kept clean for over a long time by periodically performing a defatting washing process in which, for example, oil and fat is washed away by causing an alkaline chemical, such as sodium carbonate or sodium hypochlorite, to flow through relevant flow routes included in the line section. In a known technique, a B-drug solution for preparing dialysate is heated to generate a sodium carbonate solution, and the sodium carbonate solution is used as the alkaline chemical to be caused to flow through the line section in the defatting washing process (see PTL 1, for example).

As in such a technique, if the sodium carbonate solution obtained by heating the B-drug solution, which is to be used in preparing dialysate in the dialysis treatment, is also used as the alkaline chemical in the defatting washing process, some of the B-drug solution that has remained in the dialysis treatment is reusable. Consequently, the cost of the drug is reduced. Furthermore, the number of kinds of the drugs to be used in the defatting washing process is reduced, which reduces the labor of administration.

### Citation List

### Patent Literature

PTL 1: Japanese Unexamined Patent Application Publication No. 2018-118033

### Summary of Invention

### Technical Problem

In the above known blood purification apparatus, although the B-drug solution used in the dialysis treatment (a blood purification treatment process) is reusable as the alkaline chemical (a sodium carbonate solution) in the defatting washing process, carbon dioxide (CO₂) generated by heating the B-drug solution may accumulate in the line section and hinder favorable performance of defatting washing. In the technique in which the B-drug solution is heated to generate a sodium carbonate solution to be used as an alkaline chemical in the defatting washing process, the amount of the sodium carbonate solution may become insufficient, leading to an inevitable suspension of the defatting washing process.

The present invention has been conceived in view of the above circumstances and provides a blood purification apparatus configured such that a B-drug solution used in a blood purification treatment process is also utilized as an alkaline chemical in a defatting washing process, and such that assured and smooth performance of the defatting washing process is achieved.

### Solution to Problem

A blood purification apparatus according to an embodiment of the present invention is configured to purify a patient's blood by causing the blood to extracorporeally circulate through a blood purifier and a blood circuit. The apparatus includes a line section including a dialysate introduction line through which dialysate is to be introduced into the blood purifier, and a drain-liquid discharge line through which drain liquid is to be discharged from the blood purifier; a delivery unit configured to deliver the dialysate and the drain liquid in the line section; a control unit configured to execute a blood purification treatment process and a defatting washing process, the blood purification treatment process being executed when blood purification treatment is performed in which the dialysate is delivered to the blood purifier, the dialysate being prepared by mixing an A-drug solution and a B-drug solution introduced into the line section and diluting the A-drug solution and the B-drug solution to predetermined concentrations, the defatting washing process being executed when defatting washing is performed in which oil and fat in the line section is removed by causing a bicarbonate solution to flow through the line section; a heating unit configured to heat the B-drug solution introduced into the line section; and a deaerator configured to discharge carbon dioxide to an outside of the line section, the carbon dioxide being generated from the B-drug solution heated by the heating unit. The control unit uses the heating unit and the deaerator when executing a bicarbonate-solution-generating process in which the bicarbonate solution is generated by a predetermined amount while carbon dioxide is discharged, and when executing, after the bicarbonate-solution-generating process, the defatting washing process in which the bicarbonate solution is used.

### Advantageous Effects of Invention

According to the present invention, the heating unit and the deaerator are used when the bicarbonate-solution-generating process is executed in which a bicarbonate solution is generated by a predetermined amount while carbon dioxide is discharged, and when, after the bicarbonate-solution-generating process, the defatting washing process is executed in which the bicarbonate solution is used. Therefore, while the B-drug solution used in the blood purification treatment process is utilized as an alkaline chemical in the defatting washing process, assured and smooth performance of the defatting washing process is achieved.

### Brief Description of Drawings

[Fig. 1] Fig. 1 is a schematic diagram of a blood purification apparatus according to an embodiment of the present invention.
[Fig. 2] Fig. 2 is a schematic diagram for describing a blood purification treatment process to be executed by the blood purification apparatus.
[Fig. 3] Fig. 3 is a schematic diagram for describing a bicarbonate-solution-generating process to be executed by the blood purification apparatus.
[Fig. 4] Fig. 4 is a schematic diagram for describing a defatting washing process to be executed by the blood purification apparatus.
[Fig. 5] Fig. 5 is a flow chart illustrating a control sequence to be executed by a control unit included in the blood purification apparatus.
[Fig. 6] Fig. 6 is a schematic diagram of a part of a blood purification apparatus according to another embodiment of the present invention that serves as both a heating unit and a deaerator.

### Description of Embodiments

Embodiments of the present invention will now be described specifically with reference to the drawings.

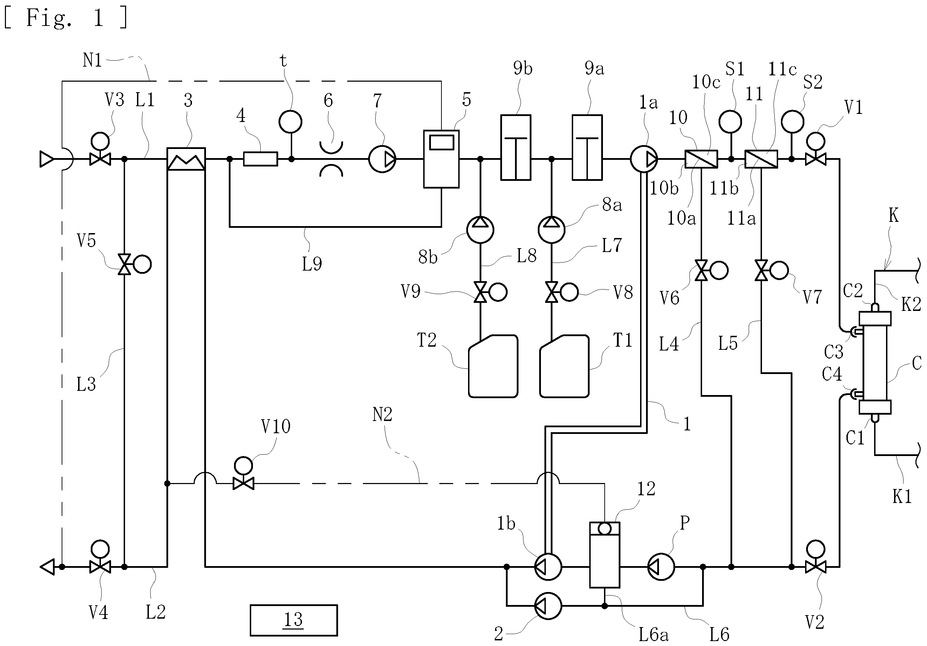

A blood purification apparatus according to an embodiment is configured to purify a patient's blood by causing the blood to extracorporeally circulate through a blood purifier and a blood circuit. As illustrated in Fig. 1, the blood purification apparatus includes a blood circuit K, which includes an arterial blood circuit K1 and a venous blood circuit K2; a dialyzer C, which serves as a blood purifier; a line section, which includes a dialysate introduction line L1 and a drain-liquid discharge line L2; a duplex pump 1, which serves as a delivery unit; and a control unit 13.

The arterial blood circuit K1 is provided with a connector at the distal end thereof. An arterial puncture needle is connectable to the arterial blood circuit K1 through the connector. The arterial blood circuit K1 is further provided with a peristaltic blood pump and an arterial air-trap chamber at respective halfway positions thereof. The venous blood circuit K2 is provided with a connector at the distal end thereof. A venous puncture needle is connectable to the venous blood circuit K2 through the connector. The venous blood circuit K2 is further provided with a venous air-trap chamber at a halfway position thereof.

The dialyzer C (the blood purifier) has, in a housing thereof, a blood inlet C1 (a blood introduction port), a blood outlet C2 (a blood delivery port), a dialysate inlet C3 (an inlet of a dialysate flow route, or a dialysate introduction port), and a dialysate outlet C4 (an outlet of the dialysate flow route, or a dialysate delivery port). The arterial blood circuit K1 is connected to the blood inlet C1. The venous blood circuit K2 is connected to the blood outlet C2. The dialysate inlet C3 and the dialysate outlet C4 are connected to the dialysate introduction line L1 and the drain-liquid discharge line L2, respectively.

The dialyzer C houses a plurality of hollow fiber membranes (not illustrated) formed of hollow fibers serving as blood purification membranes for purifying the blood. The blood purification membranes in the dialyzer C define blood flow routes (each extending between the blood inlet C1 and the blood outlet C2) through which the patient's blood flows, and dialysate flow routes (each extending between the dialysate inlet C3 and the dialysate outlet C4) through which dialysate flows. The hollow fiber membranes forming the blood purification membranes each have a number of microscopic holes (pores) extending therethrough from the outer peripheral surface to the inner peripheral surface. Impurities and the like contained in the blood are allowed to permeate through the membranes into the dialysate.

When the blood pump is activated while the patient is punctured with the arterial puncture needle connected to the distal end of the arterial blood circuit K1 and with the venous puncture needle connected to the distal end of the venous blood circuit K2, the patient's blood flows through the arterial blood circuit K1 and reaches the dialyzer C, where the blood is purified. The purified blood flows through the venous blood circuit K2 and returns into the patient's body. Thus, the patient's blood is purified by the dialyzer C while being caused to extracorporeally circulate through the blood circuit K.

The body of the apparatus includes the line section and the duplex pump 1 (the delivery unit). The line section includes the dialysate introduction line L1, through which dialysate is to be introduced into the dialyzer C; and the drain-liquid discharge line L2, through which drain liquid is to be discharged from the dialyzer C. The duplex pump 1 is configured to deliver the dialysate and the drain liquid in the line section. The duplex pump 1 includes a suction part 1a, which is connected to the dialysate introduction line L1; and a discharge part 1b, which is connected to the drain-liquid discharge line L2. That is, the duplex pump 1 is provided over the dialysate introduction line L1 and the drain-liquid discharge line L2. When the duplex pump 1 is activated, dialysate is introduced into the dialyzer C through the dialysate introduction line L1 and is then discharged from the dialyzer C through the drain-liquid discharge line L2 together with waste products and excess water in the blood. The duplex pump 1 may be replaced with any other means (such as the one including a so-called balancing chamber or the like).

As illustrated in Fig. 1, the line section including the dialysate introduction line L1 and the drain-liquid discharge line L2 further includes bypass lines L3 to L5, which are each connected to the dialysate introduction line L1 and to the drain-liquid discharge line L2; a detour line L6, which detours the discharge part 1b of the duplex pump 1 that is provided at the drain-liquid discharge line L2; and an A-drug-solution introduction line L7 and a B-drug-solution introduction line L8, through which the dialysate introduction line L1 is connected to an A-drug-solution storage tank T1 and a B-drug-solution storage tank T2, respectively.

The bypass lines L3 to L5 are provided with respective electromagnetic valves (V5 to V7). The A-drug-solution introduction line L7 and the B-drug-solution introduction line L8 are provided with respective electromagnetic valves (V8 and V9). The A-drug-solution introduction line L7 and the B-drug-solution introduction line L8 are further provided with respective infusion pumps (8a and 8b). When the infusion pump 8a is activated with the electromagnetic valve V8 being open, an A-drug solution in the A-drug-solution storage tank T1 is introduced into the dialysate introduction line L1 through the A-drug-solution introduction line L7. When the infusion pump 8b is activated with the electromagnetic valve V9 being open, a B-drug solution in the B-drug-solution storage tank T2 is introduced into the dialysate introduction line L1 through the B-drug-solution introduction line L8.

The dialysate introduction line L1 is provided with electromagnetic valves (V1 and V3), a heat exchanger 3, a heating unit 4, a deaerating pump 7, a deaerator 5, stirring chambers (9a and 9b), and traps (10 and 11). The heat exchanger 3 includes a flow route for clean water (RO water) that is supplied into the dialysate introduction line L1, and a flow route for the drain liquid that flows in the drain-liquid discharge line L2. In the heat exchanger 3, heat is exchanged between the two flow routes, whereby the heat of the drain liquid is transferred to the clean water.

The heating unit 4 is a heater attached to the dialysate introduction line L1 and is capable of heating the clean water supplied into the dialysate introduction line L1. On the downstream side (the right side in Fig. 1) relative to the heating unit 4 is provided a temperature sensor t, which is configured to detect the temperature (water temperature) of the clean water heated by the heating unit 4. On the downstream side relative to the temperature sensor t is provided an orifice 6. When the deaerating pump 7 is activated, the clean water having flowed through the orifice 6 is introduced into the deaerator 5.

The deaerator 5 is a chamber connected to the dialysate introduction line L1 and is provided at the bottom thereof with a circulatory line L9 (a circulatory flow route). The circulatory line L9 is a part of the line section and is connected to a predetermined position (a position between the heat exchanger 3 and the heating unit 4) of the dialysate introduction line L1 that is on the upstream side relative to the deaerator 5, so that the clean water supplied into the dialysate introduction line L1 is allowed to circulate therethrough. The deaerator 5 is further provided at the top thereof with a deaerating line N1. The deaerating line N1 extends up to the drain-liquid discharge line L2, so that bubbles collected by the deaerator 5 are dischargeable to the outside of the line section through the deaerating line N1.

The A-drug solution and the B-drug solution have different compositions for generating dialysate. Specifically, the A-drug solution (A-solution) is a mixed aqueous solution containing sodium chloride, potassium chloride, calcium chloride, magnesium chloride, sodium acetate, and the like. The B-drug solution (B-solution) is an aqueous solution of sodium hydrogen carbonate (NaHCO₃). The A-drug solution and the B-drug solution are stored in the A-drug-solution storage tank T1 and the B-drug-solution storage tank T2, respectively, by predetermined amounts. When the infusion pumps (8a and 8b) are activated, the A-drug solution and the B-drug solution are introduced into the dialysate introduction line L1.

The A-drug solution and the B-drug solution introduced into the dialysate introduction line L1 through the A-drug-solution introduction line L7 and the B-drug-solution introduction line L8 are mixed and diluted with clean water in the stirring chambers (9a and 9b), whereby a dialysate of predetermined concentrations is prepared. Specifically, while clean water supplied into the dialysate introduction line L1 is caused to circulate through the circulatory line L9, the clean water is heated by the heating unit 4 and is deaerated (bubbles are discharged) by the deaerator 5. The clean water thus heated and deaerated is mixed with the A-drug solution and the B-drug solution, whereby a dialysate of predetermined concentrations is obtained.

The traps (10 and 11) are configured to capture, in a blood purification treatment process, bubbles in the dialysate while allowing the dialysate to flow therethrough. The traps (10 and 11) include respective bubble filters (10a and 11a); respective first regions (10b and 11b), which are on the upstream side relative to the respective bubble filters (10a and 11a) and in which the bubbles captured are stored; and respective second regions (10c and 11c), which are on the downstream side relative to the respective bubble filters (10a and 11a) and allow the dialysate received to flow therethrough. The first region 10b of the trap 10 is connected to the drain-liquid discharge line L2 through the bypass line L4. The first region 11b of the trap 11 is connected to the drain-liquid discharge line L2 through the bypass line L5.

The dialysate introduction line L1 is further provided with fluid-pressure detectors (S1 and S2). The fluid-pressure detectors (S1 and S2) are provided on the downstream side (or the upstream side) relative to the respective bubble filters (10a and 11a) and are each a sensor capable of detecting fluid pressure. If a predetermined change in the fluid pressure is detected by either of the fluid-pressure detectors (S1 and S2) in the blood purification treatment process, a corresponding one of the electromagnetic valves (V6 and V7) (on-off valves) is opened, whereby bubbles in a corresponding one of the first regions (10b and 11b) are discharged to the outside of the line section through a corresponding one of the bypass lines (L4 and L5) and through the drain-liquid discharge line L2.

The drain-liquid discharge line L2 is provided with electromagnetic valves (V2 and V4), a pressurizing pump P, and a degassing chamber 12. The detour line L6 that detours the discharge part 1b of the duplex pump 1 is provided with an ultrafiltration pump 2. The ultrafiltration pump 2 is intended to remove water (excess water) from the patient's blood flowing through the dialyzer C. Specifically, when the ultrafiltration pump 2 is activated, the amount of drain liquid to be discharged through the drain-liquid discharge line L2 becomes greater than the amount of dialysate to be introduced through the dialysate introduction line L1. Consequently, an amount of water that corresponds to the difference is removed from the blood.

The degassing chamber 12 is a chamber connected to the drain-liquid discharge line L2, with the bottom thereof connected to a predetermined position of the detour line L6 through a connecting line L6a. The degassing chamber 12 is further connected at the top thereof to a deaerating line N2. The deaerating line N2 extends up to a downstream part of the drain-liquid discharge line L2, so that bubbles collected by the degassing chamber 12 are dischargeable to the outside of the line section through the deaerating line N2. The deaerating line N2 is provided with an electromagnetic valve V10 and is therefore openable and closable at any timing.

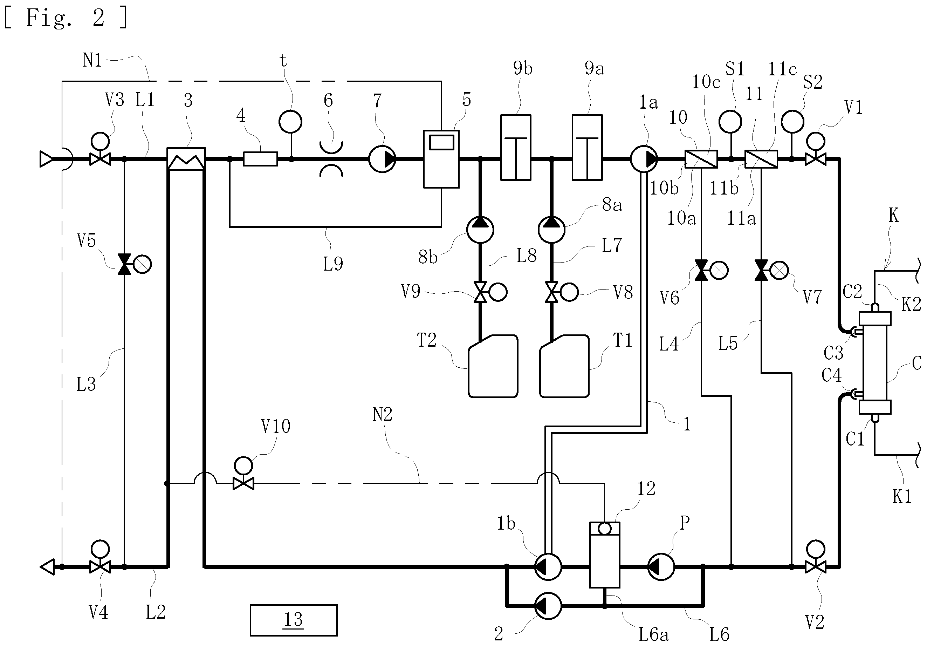

The control unit 13 is a microcomputer provided in the body of the apparatus. The control unit 13 is configured to execute the blood purification treatment process when blood purification treatment is performed, in which dialysate is delivered to the dialyzer C. The dialysate is prepared by mixing the A-drug solution and the B-drug solution introduced into the line section and diluting the A-drug solution and the B-drug solution to predetermined concentrations. Specifically, in the blood purification treatment process, as illustrated in Fig. 2, the electromagnetic valves (V8 and V9) are opened for the infusion pumps (8a and 8b) to be activated, and the duplex pump 1 and the ultrafiltration pump 2 are activated. Accordingly, the A-drug solution and the B-drug solution introduced into the dialysate introduction line L1 are mixed with clean water into a dialysate of predetermined concentrations. The dialysate thus obtained is delivered to the dialyzer C for dialysis treatment in which the patient's blood is purified and ultrafiltered.

In addition to the blood purification treatment process, the control unit 13 according to the present embodiment is capable of executing the following processes by using the heating unit 4 and the deaerator 5: a bicarbonate-solution-generating process in which a bicarbonate solution is generated by a predetermined amount while carbon dioxide is discharged; and a defatting washing process of performing defatting washing in which oil and fat in the line section is removed by causing the bicarbonate solution to flow through the line section. The bicarbonate solution is obtained by heating the B-drug solution introduced into the line section. The bicarbonate-solution-generating process is a process of generating an alkaline chemical (a sodium carbonate solution) from the B-drug solution used in the blood purification treatment process. The alkaline chemical is to be used in the defatting washing process, in which the alkaline chemical generated in the bicarbonate-solution-generating process is caused to flow through the line section, whereby defatting washing is performed.

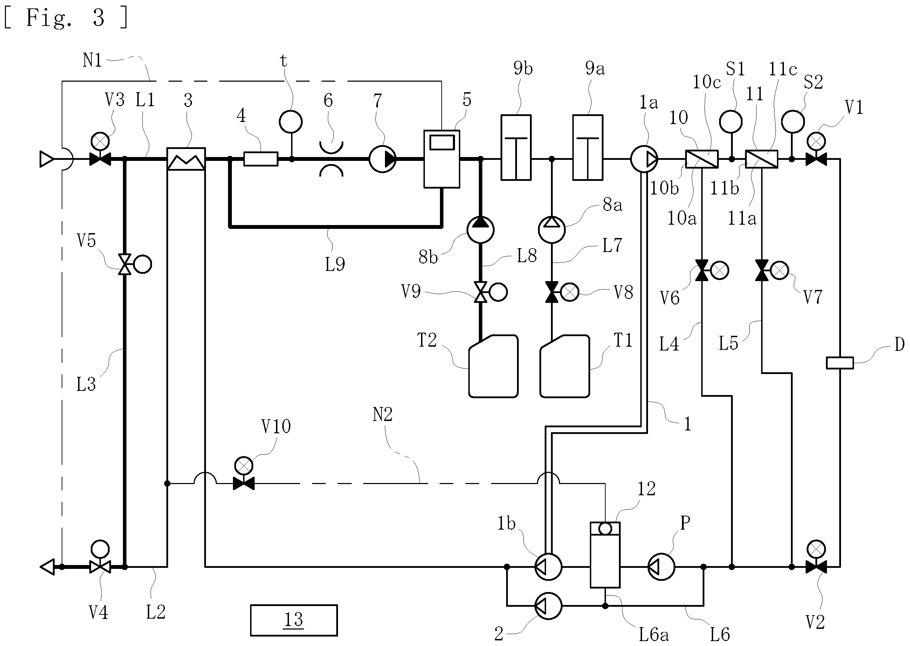

More specifically, the bicarbonate-solution-generating process is performed as follows. As illustrated in Fig. 3, the distal end of the dialysate introduction line L1 and the distal end of the drain-liquid discharge line L2 are coupled to each other with a coupler D to establish a short circuit. Furthermore, the electromagnetic valves (V4, V5, and V9) are opened, whereas the other electromagnetic valves are closed. Furthermore, the deaerating pump 7 and the infusion pump 8b are activated. In this process, devices such as the duplex pump 1 and the ultrafiltration pump 2 are stopped. Thus, the B-drug solution in the B-drug-solution storage tank T2 is introduced into the dialysate introduction line L1 and is heated by the heating unit 4 while being caused to circulate through the circulatory line L9.

While the B-drug solution (sodium hydrogen carbonate (NaHCO₃)) is circulating through the circulatory line L9, the B-drug solution is diluted and is heated (to about 65°C or above) by the heating unit 4, thereby being decomposed into sodium carbonate (Na₂CO₃), water (H₂O), and carbon dioxide (CO₂). Thus, a sodium carbonate solution (a bicarbonate solution) that serves as the alkaline chemical to be used in the defatting washing process is obtained from the B-drug solution. The carbon dioxide generated from the B-drug solution heated by the heating unit 4 is collected by the deaerator 5 and is discharged to the outside of the line section through the deaerating line N1 and the drain-liquid discharge line L2.

In the bicarbonate-solution-generating process, the heating unit 4 heats the B-drug solution introduced into the line section, and the deaerator 5 causes the carbon dioxide generated from the B-drug solution heated by the heating unit 4 to be discharged to the outside of the line section. That is, in the process of causing the B-drug solution to circulate through the circulatory line L9, while a sodium carbonate solution (a bicarbonate solution) is generated from the B-drug solution heated by the heating unit 4, carbon dioxide is discharged by the deaerator 5.

To summarize, the heating unit 4 and the deaerator 5 are used in executing the following processes. In the blood purification treatment process, the clean water for preparing a dialysate of predetermined concentrations by mixing the A-drug solution and the B-drug solution that are caused to circulate through the circulatory line L9 is heated and deaerated. Furthermore, in the bicarbonate-solution-generating process, while a sodium carbonate solution is generated by heating the B-drug solution, carbon dioxide is discharged. After the bicarbonate-solution-generating process is executed by using the heating unit 4 and the deaerator 5 to generate a sodium carbonate solution by a predetermined amount while discharging carbon dioxide, the sodium carbonate solution is used in the defatting washing process.

The present embodiment further employs a storing part configured to store by a predetermined amount the sodium carbonate solution generated by heating the B-drug solution. The storing part is a flow route in a predetermined part of the line section. In the present embodiment, the storing part includes the flow route (heat-exchanger flow route) of the heat exchanger 3. The heat-exchanger flow route is a part of the dialysate introduction line L1 where heat is exchanged with the drain-liquid discharge line L2. Specifically, the storing part is a flow route that is on the upstream side (the left side in Fig. 3) relative to the connection between the dialysate introduction line L1 and the B-drug-solution introduction line L8 and includes the circulatory line L9, the heat-exchanger flow route of the heat exchanger 3, and the bypass line L3. The storing part is capable of storing the sodium carbonate solution generated in the bicarbonate-solution-generating process.

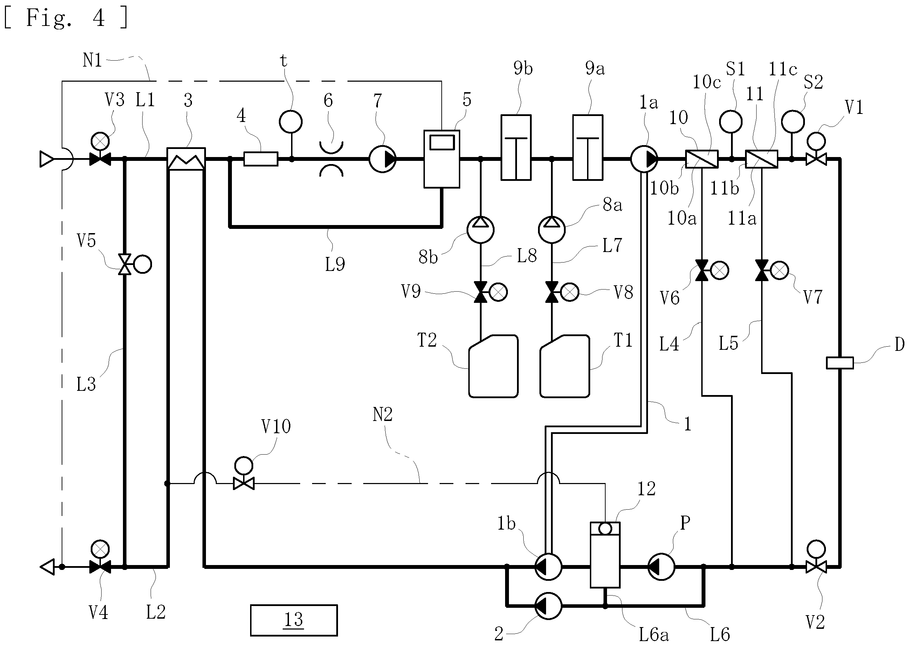

In the defatting washing process, as illustrated in Fig. 4, the distal end of the dialysate introduction line L1 and the distal end of the drain-liquid discharge line L2 are kept being coupled to each other with the coupler D. Furthermore, the electromagnetic valves (V1, V2, V5, and V10) are opened, whereas the other electromagnetic valves are closed. Furthermore, the deaerating pump 7, the duplex pump 1, the ultrafiltration pump 2, and the pressurizing pump P are activated. In this process, the infusion pumps (8a and 8b) are stopped. Note that the electromagnetic valves (V5 to V7) are opened at any timings when the bypass lines (L3 to L5) are subjected to defatting washing. Thus, the sodium carbonate solution stored in the storing part is caused to flow and circulate through the line section, whereby defatting washing is achieved.

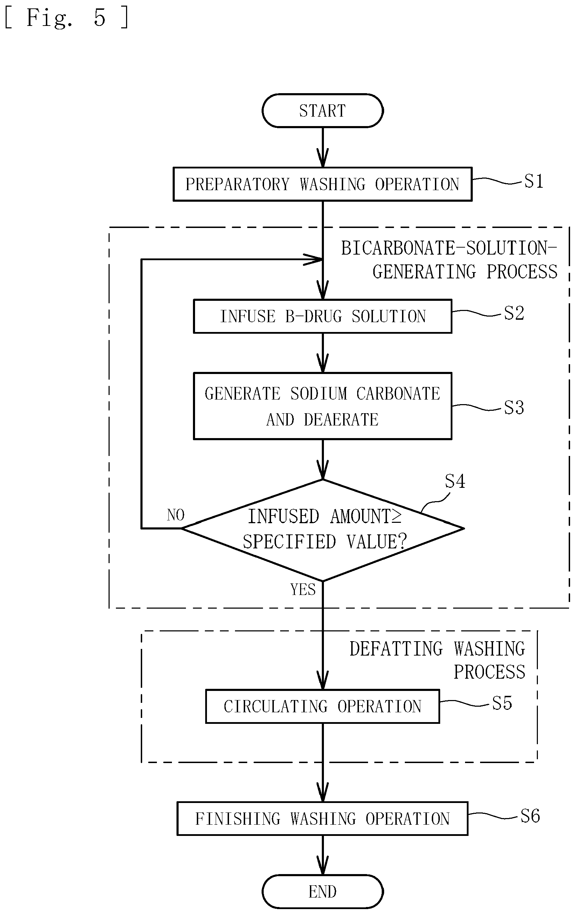

Now, a control sequence executed by the control unit 13 according to the present embodiment will be described with reference to a flow chart illustrated in Fig. 5.

First, a preparatory washing operation is performed (S1). Specifically, the distal end of the dialysate introduction line L1 and the distal end of the drain-liquid discharge line L2 are coupled to each other with the coupler D to establish a short circuit, and clean water is supplied into the dialysate introduction line L1 and is caused to flow through relevant flow routes included in the line section. Thus, the dialysate in the line section is washed away.

Subsequently, as illustrated in Fig. 3, the B-drug solution in the B-drug-solution storage tank T2 is introduced into the dialysate introduction line L1 (S2), and the bicarbonate-solution-generating process is executed. In this process, the B-drug solution is caused to circulate through the circulatory line L9 and is heated by the heating unit 4. Thus, a sodium carbonate (Na₂CO₃) solution of a predetermined concentration is generated, while carbon dioxide (CO₂) is discharged through the deaerating line N1 (S3).

The sodium carbonate solution thus generated is stored in the storing part, which is a flow route that is on the upstream side (the left side in Fig. 3) relative to the connection between the dialysate introduction line L1 and the B-drug-solution introduction line L8 and includes the circulatory line L9, the heat-exchanger flow route of the heat exchanger 3, and the bypass line L3. Then, whether the amount of the B-drug solution infused has reached a specified value or greater is checked in S4. If it is determined that the infused amount has reached the specified value or greater, the process proceeds to S5, where the defatting washing process is executed.

In the defatting washing process, as illustrated in Fig. 4, the duplex pump 1 and other relevant devices are activated, whereby the sodium carbonate solution stored in the storing part is caused to circulate through relevant flow routes in the line section (S5). In the defatting washing process, the A-drug-solution storage tank T1 and the B-drug-solution storage tank T2 may be detached from the A-drug-solution introduction line L7 and the B-drug-solution introduction line L8, so that the sodium carbonate solution may be caused to flow through the A-drug-solution introduction line L7 and the B-drug-solution introduction line L8 for defatting washing thereof. In either way, patient-originated proteins are removed by the washing effect of the sodium carbonate solution. Thus, defatting washing of the line section is achieved.

When the defatting washing process ends, a finishing washing operation is performed (S6). Specifically, clean water is supplied into the dialysate introduction line L1 and is caused to flow through relevant flow routes included in the line section. Thus, the sodium carbonate solution in the line section is washed away. Through the above operations, the bicarbonate-solution-generating process and the defatting washing process are ended. Since the line section is thus washed and disinfected, another run of the blood purification treatment process is ready to be executed.

According to the present embodiment, the heating unit 4 and the deaerator 5 are used when the bicarbonate-solution-generating process is executed in which a sodium carbonate solution is generated by a predetermined amount while carbon dioxide is discharged, and when, after the bicarbonate-solution-generating process, the defatting washing process is executed in which the sodium carbonate solution is used. Therefore, while the B-drug solution used in the blood purification treatment process is utilized as the alkaline chemical in the defatting washing process, assured and smooth performance of the defatting washing process is achieved.

Furthermore, the blood purification apparatus according to the present embodiment includes the B-drug-solution introduction line L8 through which the B-drug solution is to be introduced into the line section. The heating unit 4 and the deaerator 5 are located on the upstream side relative to the B-drug-solution introduction line L8 in the direction of delivery (the rightward direction in Fig. 3) of the sodium carbonate solution in the defatting washing process. The storing part (the flow route that is on the upstream side relative to the connection between the dialysate introduction line L1 and the B-drug-solution introduction line L8 and includes the circulatory line L9, the heat-exchanger flow route of the heat exchanger 3, and the bypass line L3) is located on the upstream side relative to the heating unit 4 and the deaerator 5 in the direction of delivery. The control unit 13 executes the bicarbonate-solution-generating process such that a bicarbonate solution is generated by heating and deaerating, with the heating unit 4 and the deaerator 5, the B-drug solution introduced from the B-drug-solution introduction line L8 in a direction opposite to the direction of delivery (the leftward direction in Fig. 3) of the bicarbonate solution, and such that the bicarbonate solution is further delivered in the direction opposite to the direction of delivery and is stored in the storing part. Therefore, the B-drug solution is heated and deaerated immediately after the introduction thereof. Furthermore, the B-drug solution of a predetermined amount is introduced continuously from the heating unit 4 and the deaerator 5 to the storing part by being caused to flow in the direction opposite to the direction of delivery.

Furthermore, the line section includes the circulatory line L9 (the circulatory flow route) through which the B-drug solution introduced into the line section is caused to circulate. In the process of causing the B-drug solution to circulate through the circulatory line L9, while a sodium carbonate solution is generated from the B-drug solution heated by the heating unit 4, carbon dioxide is discharged by the deaerator 5. Therefore, the carbon dioxide generated with the generation of the sodium carbonate is assuredly and favorably discharged to the outside of the line section.

Furthermore, in the blood purification treatment process, the clean water for preparing a dialysate of predetermined concentrations by mixing the A-drug solution and the B-drug solution that are caused to circulate through the circulatory line L9 is heated and deaerated by the heating unit 4 and the deaerator 5. That is, the heating unit 4, the deaerator 5, and the circulatory line L9 that are used in the blood purification treatment process are also utilized in the bicarbonate-solution-generating process in which the B-drug solution is heated, deaerated, and caused to circulate.

Furthermore, the blood purification apparatus includes the storing part configured to store by a predetermined amount the bicarbonate solution generated by heating the B-drug solution. Therefore, the sodium carbonate solution of an amount required in the defatting washing process is obtained in the bicarbonate-solution-generating process. In particular, the storing part is a flow route in a predetermined part of the line section. Therefore, no additional element needs to be provided to obtain the storing part. Furthermore, the storing part includes the heat-exchanger flow route of the heat exchanger 3 that is a part of the dialysate introduction line L1 and where heat is exchanged with the drain-liquid discharge line L2. Therefore, the sodium carbonate solution is storable by a relatively large amount.

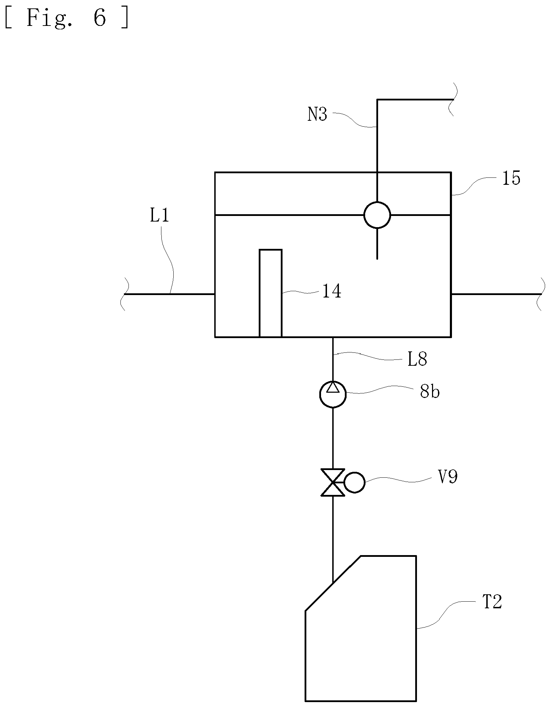

In another embodiment, as illustrated in Fig. 6, the storing part may be a container 15. The container 15 is provided with a heating unit 14 and a deaerating line N3, which serves as a deaerator. The container 15 is capable of storing by a predetermined amount the sodium carbonate solution generated through the heating of the B-drug-solution by the heating unit 14. The container 15 serving as the storing part is connected to the dialysate introduction line L1, and is also connected to the B-drug-solution storage tank T2 through the B-drug-solution introduction line L8.

The heating unit 14 is provided inside the container 15. The deaerating line N3 extends from the top of the container 15, so that any gas (carbon dioxide) in the container 15 is dischargeable to the outside. The container 15 stores the clean water supplied into the dialysate introduction line L1. When the bicarbonate-solution-generating process is executed, the infusion pump 8b is activated with the electromagnetic valve V9 being open, whereby the B-drug solution in the B-drug-solution storage tank T2 is introduced into the container 15.

Accordingly, the B-drug solution is diluted with the clean water in the container 15 and is heated by the heating unit 14, whereby a sodium carbonate (Na₂CO₃) solution of a predetermined concentration is generated, with carbon dioxide (CO₂) being discharged through the deaerating line N3. As in such an embodiment, if the storing part is a container that is provided with the heating unit 14 and the deaerating line N3 serving as a deaerator and that is configured to store by a predetermined amount the sodium carbonate solution generated through the heating of the B-drug-solution by the heating unit 14, assured generation of the sodium carbonate solution required in the defatting washing process and assured discharge of carbon dioxide are both achieved.

Furthermore, the container 15 is connected to the dialysate introduction line L1, and the heating unit 14 and the deaerating line N3 serving as a deaerator are used in the blood purification treatment process in preparing a dialysate of predetermined concentrations by mixing the clean water, the A-drug solution and the B-drug solution. That is, the container 15 is utilized in both the blood purification treatment process and the bicarbonate-solution-generating process. The container 15 does not necessarily need to be connected to the dialysate introduction line L1 and may be connected to, for example, the B-drug-solution introduction line. In that case, the container 15 is employable with no changes in the elements connected to the dialysate introduction line L1 and in the arrangement thereof.

While some embodiments have been described above, the present invention is not limited thereto. For example, the deaerating line N3 through which carbon dioxide generated in the bicarbonate-solution-generating process is to be discharged may be replaced with a line that is open to the atmosphere and through which the carbon dioxide is released to the outdoor air. Furthermore, the heating unit 4 and the deaerator 5 may each be any means that is also utilized in the blood purification treatment process as in the above embodiments, or may each be any dedicated means that is not used in the blood purification treatment process.

The above embodiments relate to a case where defatting washing of the line section is performed by causing the sodium carbonate solution obtained by heating the B-drug solution introduced into the line section to flow through the line section. Alternatively, another bicarbonate solution that is different from a sodium carbonate solution may be caused to flow. Moreover, while the above embodiment relates to a single-patient dialysis apparatus, the present invention is also applicable to any other blood purification apparatus such as a multi-patient dialysis apparatus.

### Industrial Applicability

The present invention is also applicable to a blood purification apparatus having a different appearance, additional functions, or the like, as long as the blood purification apparatus is equivalent to the spirit of the present invention.

### Reference Signs List

- 1: duplex pump (delivery unit)
- 1a: suction part
- 1b: discharge part
- 2: ultrafiltration pump
- 3: heat exchanger
- 4: heating unit
- 5: deaerator
- 6: orifice
- 7: deaerating pump
- 8a, 8b: infusion pump
- 9a, 9b: stirring chamber
- 10, 11,: trap
- 10a, 11a: bubble filter
- 10b, 11b: first region
- 10c, 11c: second region
- 12: degassing chamber
- 13: control unit
- 14: heating unit
- 15: container
- L1: dialysate introduction line
- L2: drain-liquid discharge line
- L3 to L5: bypass line
- L6: detour line
- L6a: connecting line
- L7: A-drug-solution introduction line
- L8: B-drug-solution introduction line
- L9: circulatory line
- N1, N2, N3: deaerating line
- C: dialyzer (blood purifier)
- C1: blood inlet
- C2: blood outlet
- C3: dialysate inlet
- C4: dialysate outlet
- K: blood circuit
- K1: arterial blood circuit
- K2: venous blood circuit
- T1: A-drug-solution storage tank
- T2: B-drug-solution storage tank
- t: temperature sensor
- S1, S2: fluid-pressure detector
- P: pressurizing pump

## Claims

1. A blood purification apparatus configured to purify a patient's blood by causing the blood to extracorporeally circulate through a blood purifier and a blood circuit, the blood purification apparatus comprising:
a line section including a dialysate introduction line through which dialysate is to be introduced into the blood purifier, and a drain-liquid discharge line through which drain liquid is to be discharged from the blood purifier;
a delivery unit configured to deliver the dialysate and the drain liquid in the line section;
a control unit configured to execute a blood purification treatment process and a defatting washing process, the blood purification treatment process being executed when blood purification treatment is performed in which the dialysate is delivered to the blood purifier, the dialysate being prepared by mixing an A-drug solution and a B-drug solution introduced into the line section and diluting the A-drug solution and the B-drug solution to predetermined concentrations, the defatting washing process being executed when defatting washing is performed in which oil and fat in the line section is removed by causing a bicarbonate solution to flow through the line section;
a heating unit configured to heat the B-drug solution introduced into the line section; and
a deaerator configured to discharge carbon dioxide to an outside of the line section, the carbon dioxide being generated from the B-drug solution heated by the heating unit,
wherein the control unit uses the heating unit and the deaerator when executing a bicarbonate-solution-generating process in which the bicarbonate solution is generated by a predetermined amount while carbon dioxide is discharged, and when executing, after the bicarbonate-solution-generating process, the defatting washing process in which the bicarbonate solution is used.

2. The blood purification apparatus according to Claim 1, further comprising a circulatory flow route through which the B-drug solution introduced into the line section is caused to circulate, wherein in a process of causing the B-drug solution to circulate through the circulatory flow route, while the bicarbonate solution is generated from the B-drug solution heated by the heating unit, carbon dioxide is discharged by the deaerator.

3. The blood purification apparatus according to Claim 2, wherein in the blood purification treatment process, clean water for preparing the dialysate of the predetermined concentrations by mixing the A-drug solution and the B-drug solution that are caused to circulate through the circulatory flow route is heated and deaerated by the heating unit and the deaerator.

4. The blood purification apparatus according to any of Claims 1 to 3, further comprising a storing part configured to store by a predetermined amount the bicarbonate solution generated by heating the B-drug solution.

5. The blood purification apparatus according to Claim 4, wherein the storing part is a flow route in a predetermined part of the line section.

6. The blood purification apparatus according to Claim 5, wherein the storing part includes a heat-exchanger flow route that is a part of the dialysate introduction line and where heat is exchanged with the drain-liquid discharge line.

7. The blood purification apparatus according to Claim 4, wherein the storing part is a container integrated with the heating unit and the deaerator and configured to store by a predetermined amount the bicarbonate solution generated by using the heating unit and the deaerator.

8. The blood purification apparatus according to Claim 7,
wherein the container is connected to the dialysate introduction line, and
wherein in the blood purification treatment process, clean water for preparing the dialysate of the predetermined concentrations by mixing the A-drug solution and the B-drug solution is heated and deaerated by the heating unit and the deaerator.

9. The blood purification apparatus according to Claim 7, further comprising:
an A-drug-solution introduction line through which the A-drug solution is to be introduced into the dialysate introduction line; and
a B-drug-solution introduction line through which the B-drug solution is to be introduced into the line section,
wherein the container is connected to the B-drug-solution introduction line.

10. The blood purification apparatus according to any of Claims 1 to 9, further comprising:
a B-drug-solution introduction line through which the B-drug solution is to be introduced into the line section,
wherein the heating unit and the deaerator are located on an upstream side relative to the B-drug-solution introduction line in a direction of delivery of the bicarbonate solution in the defatting washing process,
wherein the storing part is located on the upstream side relative to the heating unit and the deaerator in the direction of delivery, and
wherein the control unit executes the bicarbonate-solution-generating process such that the bicarbonate solution is generated by heating and deaerating, with the heating unit and the deaerator, the B-drug solution introduced from the B-drug-solution introduction line in a direction opposite to the direction of delivery, and such that the bicarbonate solution is further delivered in the direction opposite to the direction of delivery and is stored in the storing part.
